Europäisches Patentamt

European Patent Office

Office européen des brevets

(11) Veröffentlichungsnummer: **0 478 889 A2**

# EUROPÄISCHE PATENTANMELDUNG

(21) Anmeldenummer: **91108120.6**

(51) Int. Cl.5: **C07C 29/152**, C07C 29/78

(22) Anmeldetag: **18.05.91**

(30) Priorität: **29.09.90 DE 4030895**

(43) Veröffentlichungstag der Anmeldung:
**08.04.92 Patentblatt 92/15**

(84) Benannte Vertragsstaaten:
**CH DE DK GB IT LI**

(71) Anmelder: **Uhde GmbH**
**Friedrich-Uhde-Strasse 15 Postfach 262**
**W-4600 Dortmund 1(DE)**

(72) Erfinder: **Bähnisch, Hans-Joachim**
**Wembersweg 30**
**W-4600 Dortmund 76(DE)**

(74) Vertreter: **Patentanwälte Meinke und**
**Dabringhaus Dipl.-Ing. J. Meinke Dipl.-Ing. W.**
**Dabringhaus**
**Westenhellweg 67**
**W-4600 Dortmund 1(DE)**

(54) **Verfahren zur Verbesserung der Methanolausbeute in einer Methanolsynthese.**

(57) Es wird ein Verfahren und eine Anlage zur Verbesserung der Methanolausbeute in einer Methanolsynthese unter Einsatz eines Synthesereaktors mindestens eines Wärmeaustauschers, eines Methanolkondensators und eines Rohmethanolabscheiders angegeben, mit der Restmethanol noch gewonnen werden kann.

Dies wird dadurch erreicht, daß wenigstens ein Teil des den Synthesereaktor verlassenden Gases zu einer weiteren Wärmeabgabe einer weiteren Kondensation von Methanol und einer weiteren Methanolabscheidung zugeführt wird.

Die Erfindung richtet sich auf ein Verfahren zur Verbesserung der Methanolausbeute in einer Methanolsynthese unter Einsatz eines Synthesereaktors, mindestens eines Wärmeaustauschers, eines Methanolkondensators und eines Rohmethanolabscheiders.

Es gibt eine Reihe von Literaturstellen, die sich mit der Methanolherstellung befassen. Die nachfolgende, nach dem Anmeldetag (AT) der jeweiligen Literaturstelle geordnete Aufstellung gibt hier einen Überblick:

DE 21 17 060-A1 (AT 07.04.1971)
DE 25 29 591-A1 (AT 02.07.1975)
US 4 239 693 (AT 22.06.1979)
DE 32 20 995-A1 (AT 03.06.1982)
EP 0 067 491-B1 (AT 11.06.1982)
DE 35 18 362-A1 (AT 22.05.1985)
DE 35 25 904-A1 (AT 19.07.1985)
GB 2 214 912-A (AT 04.02.1988)
EP 0 326 718-A1 (AT 05.02.1988)
EP 0 329 292-A2 (AT 25.01.1989)
DE 40 01 713-A1 (AT 22.01.1990).

Die oben angegebenen Literaturstellen beschäftigen sich mit vielfältigen Problemen, so mit der Optimierung der Energieausbeute, dank vereinfachter Verfahrensführung und auch mit Optimierung der Produktausbeute.

Aufgabe der Erfindung ist die Schaffung einer Lösung, mit der die Methanolausbeute erhöht werden kann. Mit einem Verfahren der eingangs bezeichneten Art wird diese Aufgabe gemäß der Erfindung dadurch gelöst, daß wenigstens ein Teil des den Synthesereaktor verlassenden Gases zu einer weiteren Wärmeabgabe einer weiterer Kondensation von Methanol und einer weiteren Methanolabscheidung zugeführt wird.

Die Erfindung macht sich die Erkenntnis zunutze, daß sich im Gas hinter dem Rohmethanol-Abscheider im Synthesekreislauf noch ca. 0,5 mol% Methanol befinden. Dieses Methanol wird erfindungsgemäß durch eine nachgeschaltete, zweite Stufe der Behandlung gewonnen, und zwar durch eine weitere Methanolkondensation und Methanolabscheidung.

Durch die erfindungsgemäße Verfahrensweise erhöht sich die Produktion einer Methanolanlage, um beispielsweise bis zu 15 %, wobei der Restmethanolanteil bei ca. 0,05 % liegt. Es versteht sich von selbst, daß der Anstieg der Ausbeute abhängig ist vom Synthesegas sowie von den Abscheidebedingungen, wie Druck und Temperatur.

Grundsätzlich ist es natürlich möglich, wie auch im Stand der Technik beschrieben, bei der Methanolsynthese Kühlwasser zur Kondensation des Produktes aus dem Kreislauf auszukondensieren. Die Erfindung richtet sich aber auf die verbleibenden, vergleichsweise geringen Methanolanteile aus dem Reaktionsgasstrom nach dem Rohmethanolabscheider, die mit Kühlwasser nicht mehr kondensierbar sind. Durch die erfinderische Ausgestaltung, nämlich daß die weitere Kondensation des Gases durch indirekten Wärmeaustausch in einem geschlossenen Kältekreislauf betriebenen Kältemittel erfolgt, lassen sich die oben beschriebenen weiteren Vorteile erreichen.

Die Erfindung sieht auch vor, daß nach der weiteren Methanolabscheidung das Gas im indirekten Wärmeaustausch zu dem die erste Methanolabscheidung verlassenden Gas erwärmt wird. Erkennbar optimiert eine solche Vorgehensweise die energetische Bilanz.

Zweckmäßig kann es sein, wenn nach der indirekten Erwärmung und nach einem Abzug von Purgegas eine Zumischung von Frischgas erfolgt und dieser Gasstrom einer Kreislaufverdichtung zugeführt wird, wie dies die Erfindung ebenfalls in weiterer Ausgestaltung vorsieht.

In vorteilhafter weiterer Ausgestaltung ist nach der Erfindung auch vorgesehen, daß die die erste und zweite Rohmethanolabscheidung verlassenden flüssigen Ströme einer gemeinsamen Druckentspannung zugeführt werden, in der eine Trennung in Entspannungsgas und Rohmethanol erfolgt.

Um die Leistung zu erhöhen kann auch vorgesehen sein, daß die durch den Kältekreislauf zur Verfügung stehende Kältemenge wenigstens teilweise für eine Frischgasverdichtung im in einer Vor- und Zwischenkühlung und/oder für eine Sekundärkondensation in der Destillation eingesetzt wird. Vorgesehen sein kann auch, daß die durch den Kältekreislauf zur Verfügung stehende Kältemenge wenigstens teilweise für eine Frischgasverdichtung in einer Vor- und Zwischenkühlung und/oder für eine Sekundärkondensation in der Destillation eingesetzt wird.

Zur Lösung der weiter oben angegebenen Aufgabe sieht die Erfindung auch eine Anlage vor, die sich dadurch auszeichnet, daß zusätzlich zu einem Synthesereaktor, einem ersten Wärmeaustauscher, einem Methanolkondensator und einem Rohmethanolabscheider in Strömungsrichtung des Synthesegases hinter dem ersten Abscheider ein von einem Kältemittelkreislauf beaufschlagter Kälte-Wärmeaustauscher (Chiller) und nachfolgend ein zweiter Rohmethanolabscheider angeordnet ist.

Die oben zur Verfahrensweise angegebenen Vorteile gelten in gleicher Weise für die hier angegebene Anlage, die sich in weiterer Ausgestaltung dadurch auszeichnet, daß vor dem Kälte-Wärmeaustauscher ein Vorkühler vorgesehen ist, wobei dieser Vorkühler von dem den zweiten Rohmethanolabscheider verlassenden Gas im Wärmeaustausch beaufschlagt ist.

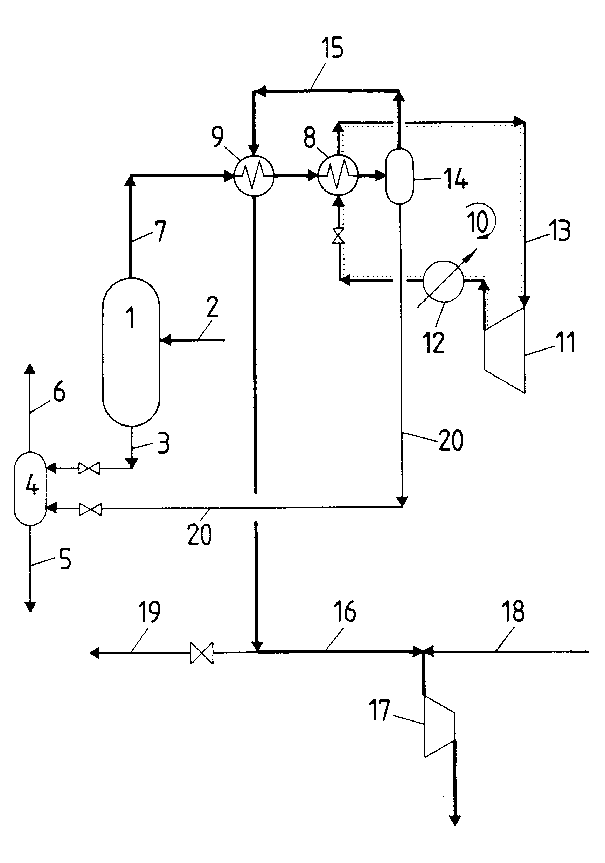

Die Erfindung ist nachstehend anhand der Zeichnung beispielsweise näher erläutert. Diese zeigt in der einzigen Figur ein Prinzipschaltbild einer Anlage bzw. Verfahrensschaltung nach der Erfindung.

Es sei bemerkt, daß es hier nur auf einen speziellen Teil einer Methanolanlage ankommt, so daß hier nur der entsprechende Ausschnitt des Anlageschaltbildes gezeigt ist.

In einem mit 1 bezeichneten Rohmethanolabscheider in einem nicht nur andeutungsweise wiedergegebenen Synthesekreislauf tritt über eine Leitung 2 Reaktionsgas nach einem nicht dargestellten Wasserkühler ein. Das Rohmethanol wird über die Leitung 3 in einem mit 4 bezeichneten Entspannungsgefäß zugeführt und über die Leitung 5 abgeleitet, wobei die Entspannungsgase über eine Leitung 6 abgezogen werden.

Das Gas aus dem Abscheider 1 wird über eine Leitung 7 einem Kälte/Wärmeaustauscher (Chiller) 8 zugeführt und dabei vorab über einen Vorkühler 9 geleitet. Dieser Kälte/Wärmeaustauscher ist integraler Bestandteil einer allgemein mit 10 bezeichneten Kälteanlage, die neben dem Chiller 8 einen Kältemittelkompressor 11 und einen Kältemittelkondensator 12 in dem mit 13 bezeichneten Kältemittelkreislauf aufweist.

Das über den Chiller 8 geleitete Synthesegas wird einem weiteren Abscheider 14 zugeführt. Das aus dem Abscheider 14 stammende Gas wird über eine Leitung 15 dem obengenannten Vorkühler 9 zur Vorkühlung des Gases aus dem ersten Abscheider zugeführt, wobei es als Kreislaufgas über eine Leitung 16 einem Kreislaufgasverdichter 17 zugeführt wird. In Bezug auf den Gaskreislauf ist in der Figur noch eine Zuführleitung 18 für die Zuführung von Synthesefrischgas eingezeichnet und eine Abführleitung 19 für Purgegas.

Das aus dem zweiten Rohmethanolabscheider 14 stammende Rohmethanol wird über eine Leitung 20 dem bereits eingangs beschriebenen Entspannungsgefäß zugeführt.

Setzt man als Beispiel eine Anlage an mit einem Ausstoß von etwa 812.000 Nm³/h als Gas aus dem ersten Abscheider 1 über die Leitung 7 bei einer Temperatur von 40° C und einer Abkühlung auf 10° C mit einem MeOH/Gehalt im Gas von 0,510 mol% und einem angenommenen Gehalt von ungefähr 0,050 % hinter der zweiten Methanolabscheidung ergibt sich eine Ausbeute von 534 200 kg/h MeOH, d.h. von 128 tato bei einer Anlage mit 1000 tato im Sinne einer Praxis eingesetzt wird.

**Patentansprüche**

1.  Verfahren zur Verbesserung der Methanolausbeute in einer Methanolsynthese unter Einsatz eines Synthesereaktors, mindestens eines Wärmeaustauschers, eines Methanolkondensators und eines Rohmethanolabscheiders, dadurch gekennzeichnet,
    daß wenigstens ein Teil des den Synthesereaktor verlassenden Gases zu einer weiteren Wärmeabgabe einer weiteren Kondensation von Methanol und einer weiteren Methanolabscheidung zugeführt wird.

2.  Verfahren nach Anspruch 1,
    dadurch gekennzeichnet,
    daß die weitere Kondensation des den Synthesereaktor verlassenden Gases durch indirekten Wärmeaustausch mit einem in einem geschlossenen Kältekreislauf betriebenen Kältemittel erfolgt.

3.  Verfahren nach Anspruch 1 oder 2,
    dadurch gekennzeichnet,
    daß nach der weiteren Methanolabscheidung das Gas im indirekten Wärmeaustausch zu dem die erste Methanolabscheidung verlassenden Gas erwärmt wird.

4.  Verfahren nach Anspruch 3,
    dadurch gekennzeichnet,
    daß nach der indirekten Erwärmung und nach einem Abzug von Purgegas eine Zumischung von Frischgas erfolgt und dieser Gasstrom einer Kreislaufgasverdichtung zugeführt wird.

5.  Verfahren nach einem der Ansprüche 1 bis 4,
    dadurch gekennzeichnet,
    daß die die erste und zweite Rohmethanolabscheidung verlassenden flüssigen Ströme einer gemeinsamen Druckentspannung zugeführt werden, in der eine Trennung in Entspannungsgas und Rohmethanol erfolgt.

6.  Verfahren nach einem der vorangehenden Ansprüche,
    dadurch gekennzeichnet,
    daß die durch den Kältekreislauf zur Verfügung stehende Kältemenge wenigstens teilweise für eine Frischgasverdichtung in einer Vor- und Zwischenkühlung und/oder für eine Sekundärkondensation in der Destillation eingesetzt wird.

7.  Verfahren nach einem der vorangehenden Ansprüche,
    dadurch gekennzeichnet,
    daß zur Aufbringung der Antriebsenergie des Kältemittelkompressors im Kältemittel eine Purgegasentspannungsturbine ganz oder teilweise eingesetzt wird.

8.  Anlage zur Verbesserung der Methanolausbeute in einer Methanolsynthese, insbesondere zur Durchführung des Verfahrens nach einem der vorangehenden Ansprüche,

dadurch gekennzeichnet,
daß zusätzlich zu dem einen Synthesereaktor, einem ersten Wärmeaustauscher, einem Methanolkondensator und einem Rohmethanolabscheider (1) in Strömungsrichtung des Synthesegases hinter dem ersten Abscheider (1) ein von einem Kältemittelkreislauf (10) beaufschlagter Kälte-Wärmeaustauscher (Chiller) (8) und nachfolgend ein zweiter Rohmethanolabscheider (14) angeordnet ist.

9. Anlage nach Anspruch 8,
dadurch gekennzeichnet,
daß vor dem Kälte-Wärmeaustauscher (8) ein Vorkühler (9) vorgesehen ist, wobei dieser Vorkühler (9) von dem den zweiten Rohmethanolabscheider (14) verlassenden Gas im Wärmeaustausch beaufschlagt ist.